# EUROPEAN PATENT SPECIFICATION

(11) **EP 0 866 049 B1**
(45) Date of publication and mention of the grant of the patent: **19.11.2003**
(21) Application number: 98301951.4
(22) Date of filing: 16.03.1998
(51) Int. Cl.: C07C 37/62, B01J 31/02, C07C 321/00, C07C 39/28

(54) **Chlorination of aromatic compounds and catalysts therefor**
Chlorierung von aromatischen Verbindungen und Katalysatoren dafür
Chloration de composés organiques et catalyseurs à cet effet

(30) Priority: 17.03.1997 GB 9705502
(43) Date of publication of application: 23.09.1998
(73) Proprietor: The University of Wales Swansea, Swansea SA2 8PP (GB)
(72) Inventor: Tzimas, Michael, 67283 Obrigheim (DE); Smith, Keith, Swansea SA3 5DT (GB); Brown, Christopher Martin, Warrington, Cheshire WA5 5UT (GB); Payne, Keith, Accrington, Lancashire BB5 5PQ (GB)
(74) Representative: Linn, Samuel Jonathan

(56) References cited:
- EP-A- 0 299 892
- GB-A- 2 177 396
- US-A- 3 920 757
- US-A- 4 160 114
- US-A- 4 564 714
- DATABASE REGISTRY CAS (STN) RN = 172534-42-8, 172534-41-7, XP002074363

## Description

The present invention relates to the chlorination of aromatic compounds, particularly phenols, using certain sulphur-containing organic compounds as catalysts. The invention also relates to certain such sulphur-containing catalyst compounds per se.

It is known, from US-A-4564714, to chlorinate selectively 2,5-dichlorophenol with sulphuryl chloride in the presence of diphenyl sulphide and sulphuric acid at a temperature of from -10° to +25°C in order to obtain 2,4,5-trichlorophenol.

More generally, regioselective mono-chlorination of phenols with sulphuryl chloride has been known since 1866, when DuBois demonstrated the treatment of molten phenol with an equimolar amount of sulphuryl chloride [Z.F.Chem. 705(1866)]. Modern analytical techniques have shown that the reaction is not as selective as was thought by DuBois, with p-chlorophenol actually being the predominantly favoured product. More recently, the catalysts of this reaction by a combination of particular divalent sulphur compounds and metal halides has been disclosed in US-A-3920757 (Watson). One of the most preferred catalysts in this document is diphenyl sulphide, in combination with AlCl₃, and this catalyst has been applied to a number of further chlorination reactions by sulphuryl chloride, giving a majority of para-mono-chlorinated product over the corresponding ortho-mono-chlorinated product.

This known catalyst system however has several disadvantages, particularly when intended for use on an industrial scale. For instance, product yields are not as great as may often be desired, coupled with the fact that only limited para:ortho chlorinated product ratios have hitherto been obtainable. (The para-mono-chlorinated products are generally the more useful industrially and are therefore preferred.) Also, the undesirable isomers and polychlorinated products hinder purification and can be costly to dispose of.

Another problem is that the known catalysts are difficult to separate from the reaction products mixture. Furthermore, the presence of AlCl₃ as a co-catalyst may be disadvantageous in that it hydrolyses on contact with water to produce acidic products which promote corrosion of metal reaction vessels and equipment. Many of these known sulphur-containing compounds also have a strong characteristic sulphurous odour, which necessitates more careful and expensive handling and equipment if health and safety hazards are to be avoided and worker-friendliness is to be optimised. Also, these known sulphur-containing catalysts are generally not re-usable, which may lead to problems of safe and environmentally friendly disposal as well as of course being detrimental to the economics of the overall process.

The present invention aims to ameliorate at least some of the above disadvantages of the prior art by providing new sulphur-containing catalysts which are useful for catalysing the chlorination reaction of phenols using a chlorinating agent, which new catalysts not only give good product yields and high para-chlorinated product:orthochlorinated product (para:ortho) ratios, but may also be substantially odourless and may be easily recovered, hence leading to improved economics of the process when applied industrially.

In a first aspect the present invention provides a process for the chlorination of an aromatic compound of the following formula (A): 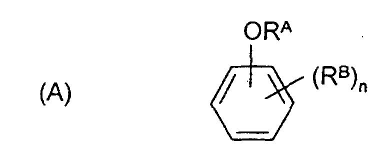 wherein R^{A} is H or C₁₋C₁₂ alkyl, cycloalkyl, aryl alkaryl, aralkyl or carboxyalkyl, the or each R^{B} independently is selected from H, C₁-C₄ alkyl (especially methyl), C₁-C₄ haloalkyl or polyhaloalkyl (e.g. C₁-C₄ perfluoroalkyl), C₁-C₄ alkoxy, C₅-C₁₂ aryl (e.g. phenyl), alkaryl or aralkyl, or halogen, n is an integer which is 0, 1 or 2, and the or each R^{B}, if present, may independently be attached at the ortho or the meta position, preferably at the meta-position, the said process comprising reacting the aromatic compound with a chlorinating agent in the presence of a sulphur-containing catalyst, optionally also in the presence of a Lewis acid co-catalyst of the formula MXₘ, where: M is a metal or metalloid such as B, Al, Ga, In, Tl, Ge, Sn, Cd, Ni, Fe, Zn, Ti, Hg, La; X is an electronegative group such as F, Cl, Br, I, C₁-C₄ alkoxide, aryloxide (e.g. phenoxide), carboxylate (e.g. acetate), arenecarboxylate (e.g. benzoate), substituted carboxylate (e.g. trifluoroacetate), C₁-C₄ alkanesulphonate, arenesulphonate or substituted sulphonatet (e.g. trifluoromethanesulphonate); and m is an integer which is preferably 1, 2, 3 or 4;
characterised in that the sulphur-containing catalyst is a compound according to the following formula (II): 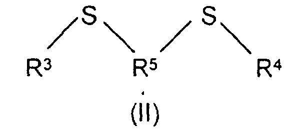 in which:
R³ and R⁴ are each independently selected from the group consisting of: H, optionally substituted straight or branched chain alkyl or alkanediyl having from 1 to 20 carbon atoms, optionally substituted straight or branched chain alkaryl or aralkyl having from 5 to 20 carbon atoms, and optionally substituted aryl having from 5 to 20 carbon atoms; and
R⁵ is an optionally substituted straight or branched chain alkylene, arylene, alkarylene or arylalkylene group having from 4 to 20 carbon atoms;
   wherein in the above definitions R³, R⁴ and R⁵ the optional substituents may be independently selected from the following: halogen (e.g. F, Cl), hydroxy, amino, cyano, nitro, C₁-C₄ alkyl, C₁-C₄ haloalkyl e.g. C₁-C₄ perfluoroalkyl, C₁-C₄ alkoxy, C₁₋C₄ alkoxycarbonyl;
   provided that when R⁵ is a C₄ alkylene group, a Lewis acid co-catalyst of the formula MXₘ (as defined above) is present.

To our knowledge, certain dithiaalkane compounds disclosed herein are novel and the invention provides according to a second aspect, a dithiaalkane compound which is:

| | |
|---|---|
| 2,10-dithiaundecane | (8) |
| 2,15-dithiahexadecane | (11) |
| 5,13-dithiaheptadecane | (15) |
| 5,14-dithiaoctadecane | (16) |
| 5,18-dithiadocosane | (18) |
| 6,13-dithiaoctadecane | (21) |
| 8,15-dithiadocosane | (22) |
| 3,16-dithiaoctadecane | (23) |
| 4,17-dithiaeicosane | (24) |
| 6,19-dithiatetracosane | (25) |
| 7,20-dithiahexacosane | (26) |
| 8,21-dithiaoctacosane | (27) |
| 2,7-dithiaundecane | (30) |
| 2,2-dimethyl-3,8-dithiadodecane | (32) |
| 1-phenyl-2,7-dithiaundecane | (33) |
| 1-phenyl-1,6-dithiadecane | (34) |
| 1-phenyl-8-methyl-1,6-dithianonane | (35) |
| 1,7-diphenyl-1,6-dithiaheptane | (36) |
| 5,12-dit-hianonadecane | (37) |
| 1-phenyl-2,9-dithiatridecane | (38) |

According to a third aspect, the present invention provides the use of a sulphur-containing compound of the formula (II), given and defined above, as a catalyst in the chlorination of an aromatic compound of formula (A) above by a chlorinating agent, e.g. sulphuryl chloride.

The above primary aspects of the invention, and preferred features and embodiments thereof, will now be described in further detail.

### The chlorinating agent and the reaction conditions of the chlorination process

In the process according to the first aspect of the invention, the chlorinating agent is suitably sulphuryl chloride (SO₂Cl₂). Other known chlorinating agents however, may be suitable, e.g. chlorine gas. The process using sulphuryl chloride with the sulphur-containing catalyst, and with the optional presence of the Lewis acid co-catalyst, is preferably carried out in a homogeneous liquid phase, without the presence of a solvent. However, a solvent may be used, if desired or necessary. Suitable solvents include alkanes such as n-hexane, THF, diethyl ether, halogenated hydrocarbons such as perchloroethylene or carbon tetrachloride, chloroform or dichloromethane, petrol ether, alcohols such as ethanol or methanol, water, pyridine, dimethylformamide, dimethylsulphoxide or the catalysts themselves, or mixtures of any of the aforesaid solvents. If a solvent is used, the temperature at which the reaction is carried out is generally lower than when there is no solvent present, e.g. if phenol is the aromatic compound to be chlorinated, then in the absence of solvent the reaction mixture may solidify at a temperature less than 35°C, which sets a practical lower limit on the possible temperature for the reaction.

For maximum selectivity it is preferable that the temperature used is as low as possible, e.g. even below 35°C, more preferably below about 20 or 25°C, even possibly down to about 0°C, but the reaction may however still work, in certain embodiments, at temperatures of as high as 85°C or more. Often the reaction will be carried out approximately at or near room temperature, e.g. in the region of about 15 to about 30°C, in order to minimise the external heating or cooling that is required.

The amount of sulphur-containing catalyst present in the reaction mixture is preferably between about 0.2 and about 10 mol%, with respect to the amount of the aromatic compound, but in the case where the catalyst acts as the solvent, it is preferably present in large molar excess (e.g. at least 500 mol% excess, preferably at least 1000 mol% excess).

The amount of sulphuryl chloride (SO₂Cl₂) (or other chlorinating agent) present in the reaction mixture may also vary, e.g. depending on the amount of aromatic compound to be reacted and whether a solvent is used. Preferably the SO₂Cl₂ is present in small excess with respect to the amount of the aromatic compound, e.g. up to about 100 mol% excess. If desired or necessary, it may be possible for the SO₂Cl₂ to be present in molar deficit, e.g. up to about 20 mol% deficit. The most preferred amount of SO₂Cl₂ present in the reaction mixture is approximately at a 2 to 20 mol% excess over the amount of aromatic compound.

The chlorination reaction may be carried out by the slow addition of the SO₂Cl₂ to a reaction mixture consisting of or containing the aromatic compound, the sulphur-containing catalyst, the Lewis acid co-catalyst (if present) and the solvent (if present). Following the addition a period of stirring is generally used to ensure that the reaction is substantially complete, whilst minimising cost.

The optional presence of the above defined Lewis acid co-catalyst in the reaction mixture may further increase the para:ortho product ratio and also the yield of the mono-chlorinated product. However, in certain industrial processes the presence of such a Lewis acid may cause a problem as regards corrosion of equipment hardware and in its separation from effluent streams, so in certain practical embodiments of the process of the invention the use of a Lewis acid co-catalyst may be less desirable. A metal halide such as AlCl₃ is often the most selective and thus preferable Lewis acid, although others may perform reasonably well. Both FeCl₃ and ZnCl₂ for instance have fewer practical and environmental disadvantages than AlCl₃ for large scale use. When used, the amount of Lewis acid in the reaction mixture (in relation to the amount of the aromatic compound) may vary between approximately 0.1 and 15 mol%, with the preferred amount being about at a 1 to 40 mol% excess over the amount of sulphur-containing catalyst used.

The scale of the chlorination process may vary, one advantage of the invention being that having a larger scale process may not deleteriously affect the para:ortho product ratio or the overall yield of the desired product from the reaction.

The sulphur-containing catalyst is a compound of the formula (II), given and defined above. Such compounds generally will have a relatively high molecular weight and hence will generally produce catalysts having less sulphurous odour and which are easier to separate from the reaction products mixture by virtue of their generally higher boiling points.

In the above formula (II), preferably R⁵ is an unsubstituted alkanediyl group, and more preferably it is straight chained. In compounds of formula (II) in which R⁵ is an unbranched, unsubstituted alkanediyl group, it is preferred that it contains from 4 to 12 carbon atoms, most preferably from 7 to 12 carbon atoms.

The groups R³ and R⁴ may have either the same or different carbon atom frameworks. In compounds of formula (II) in which R³ and R⁴ have the same carbon atom framework, both groups may be unbranched and/or unsubstituted. Preferably, both groups are unbranched and unsubstituted and independently contain from 1 to 7 carbon atoms.

In compounds of formula (II) in which R³ and R⁴ have different carbon atom frameworks, R³ is preferably selected from the group consisting of: H, optionally substituted straight or branched chain alkyl or alkanediyl having from 1 to 20 carbon atoms and optionally substituted aryl having from 5 to 20 carbon atoms, and R⁴ is different from R³ and is preferably selected from the group consisting of: optionally substituted straight or branched chain alkyl or alkanediyl having from 1 to 20 carbon atoms and optionally substituted straight or branched chain alkaryl or aralkyl having from 5 to 20 carbon atoms. Both R³ and R⁴ may be unsubstituted, and preferably R³ is selected from H, unbranched C₁ - C₄ alkyl and phenyl, and R⁴ is selected from C₄ to C₇ alkyl, phenyl and benzyl.

Symmetrical dithia- compounds of formula (II) may be synthesised by any one of the following three exemplary methods (Methods A, B and C), of which Method C can also be used to synthesise the unsymmetrical dithia- compounds of formula (II). For the sake of simplicity, the three methods are exemplified below with reference to simple dithia-alkanes only, but analogous methods can be readily applied to more complicated analogous compounds also of formula (II), as the person skilled in the art will readily appreciate.

### Method A

In this method one mole equivalent of dithiol of the formula HS-R⁵-SH is lithiated, e.g. with BuLi at -78°C in THF, and is then reacted with two mole equivalents of bromoalkane of formula Br-R³ to produce the required symmetrical dithia-alkane, R³-S-R⁵-S-R³, wherein R⁴ in the general formula (II) is the same as R³. This synthesis can be represented by the following equation:

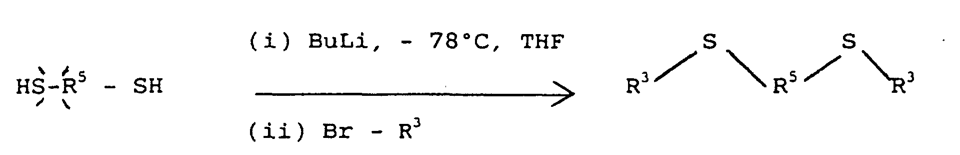

### Method B

In this method two mole equivalents of thiol of the formula HS-R³ are lithiated, e.g. with BuLi at -78°C in THF, and are then reacted with dibromoalkane of the formula Br-R⁵-Br to produce the required symmetrical dithia-alkane, R³-S-R⁵-S-R³, wherein R⁴ in the general formula (II) is the same as R³. This synthesis can be represented by the following equation:

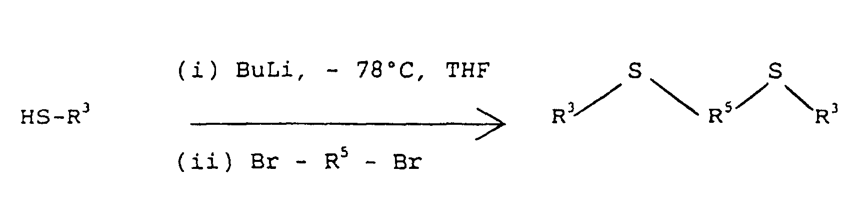

### Method C

In this method a cyclic disulphide (see below) of the formula 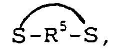 is first reacted with one equivalent of an organolithium reagent of the formula R³-Li, e.g. at -78°C in THF for 30 minutes, to produce an intermediate of the formula R³-S-R⁵-S-Li. This intermediate is then trapped with an electrophile of the formula R⁴-L, wherein L is any suitable leaving group such as halide (e.g. Cl⁻, Br⁻) or carboxylate or sulphonate, under appropriate conditions e.g. at room temperature for 12 hours, to produce the required dithia-alkane of the formula R³-S-R⁵ -S-R⁴. This synthesis can be represented by the following equation:

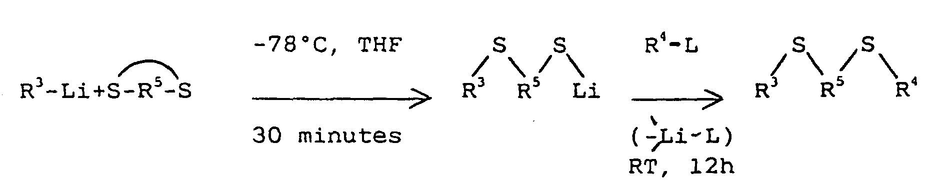

Cyclic disulphides, and more particularly 1,2-dithiacycloalkanes of ring sizes from 5 to 12, for use in Method C above may be readily synthesised by oxidative cyclisation of the readily available dithiols according to well known literature procedures, e.g. using as reagents iodine and triethylamine in trichloromethane, which reaction can be represented by the following equation:

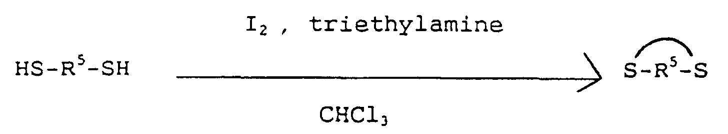

### EXAMPLES

Preferred features and embodiments of the present invention in its various aspects will now be illustrated in detail by way of the following Examples.

The sulphuryl chloride used in the following Examples is freshly distilled and is prepared by placing sulphuryl chloride in a double-necked, round-bottom flask with a few anti-bumping granules. The flask is set up for distillation and fitted with a nitrogen inlet and a water-cooled condenser leading to a three way receiver and a nitrogen outlet. The gas flows out through a scrubber. The system is flushed with nitrogen and then the gas flow is halted. The flask is as heated and the fraction boiling at 67-69°C is collected as sulphuryl chloride. This colourless liquid is stored under nitrogen.

In order to analyse the purified reaction products, a precisely known amount of the stored sample (approximately 500 mg) and 100 mg tetradecane as internal standard are weighed into a flask and diluted with 25 ml dichloromethane. 1 µl of the resulting solution is injected onto a gas chromatograph for analysis.

The gas chromatography system used involves a Philips PU 4400 instrument with a PU 4920 data station providing control, data storage and manipulation for the gas chromatograph. The conditions set for analysis are:

| | |
|---|---|
| column | 15 m carbowax megabore, ID 0.54 mm, 1.2 µm film thickness |
| carrier gas | 5 ml/min helium |
| make up gas | 25 ml/min nitrogen |
| injector temperature | 300°C |
| detector temperature | 300°C (F.I.D.) |
| injection technique | splitless |
| initial time | 2 minutes |
| column start temperature | 35°C |
| ramp rate | 20°C/min |
| upper temperature | 240°C |

The trace produced by the gas chromatograph is converted into mol% of product present in the purified reaction products using standard calculations as are well known and commonly applied in the art.

### Synthesis Examples

The following are the detailed methods for synthesising the dithia-alkanes used in Examples 19 to 22 which follow further below.

Method A: The alkanedithiol ( HS-R⁵-SH) is placed in a 100ml flame-dried round bottom flask which is then fitted with a septum and flushed with N₂. Dry THF (20ml) is added via a dry syringe and the mixture is cooled to -78°C (cardice/acetone). Butyllithium is added via a dry syringe over 20 minutes and the mixture is stirred at -78°C for another 20 minutes. The reaction is allowed to warm up to ambient temperature. The bromoalkane (Br-R³) is added via a syringe and the reaction is stirred overnight at room temperature. The reaction mixture is then concentrated under reduced pressure (from a water pump) at 60°C and the residue is dissolved in a water-diethyl ether mixture (10 ml, 1:1). The layers are separated and the water layer is washed with diethyl ether (2 x 15 ml). The organic layers are combined and dried over MgSO₄ overnight. The solution is filtered and concentrated under reduced pressure. The residue is purified using a Kugelrohr (bulb to bulb) distillation.

Method B: The alkanethiol (HS-R³) is placed in a 100 ml dried round bottom flask which is then fitted with a septum and flushed with N₂. Dry THF (20 ml) is added via a dry syringe and the mixture is cooled to -78°C (cardice/acetone). Butyllithium is added via a dry syringe over 20 minutes and the mixture is stirred at -78°C for another 20 minutes. The reaction is allowed to warm up to ambient temperature. The dibromoalkane (Br-R⁵-Br) is added via a syringe and the reaction is stirred overnight at room temperature. The reaction mixture is then concentrated under reduced pressure (from a water pump) at 60°C and the residue is dissolved in a water-diethyl ether mixture (10ml, 1:1). The layers are separated and the water layer is washed with diethyl ether (2 x 15 ml). The organic layers are combined and dried over MgSO₄ overnight. The solution is filtered and concentrated under reduced pressure. The residue is purified using a Kugelrohr (bulb to bulb) distillation.

Method C: The cyclic disulphide ( 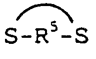 ) (see below) is placed in a 100 ml dried round bottom flask which is then fitted with a septum and flushed with N₂. Dry THF (20 ml) is added via a dry syringe and the mixture is cooled to -78°C (cardice/acetone). The organolithium reagent (R³-Li) is added via a dry syringe over 20 minutes and the mixture is stirred at -78°C for another 20 minutes. The reaction is allowed to warm up to ambient temperature. The electrophile (R⁴-L) is added via a syringe and the reaction is stirred overnight at room temperature. The reaction mixture is then concentrated under reduced pressure (from a water pump) at 60°C and the residue is dissolved in a water-diethyl ether mixture (10 ml, 1:1). The layers are separated and the water layer is washed with diethyl ether (2 x 15 ml). The organic layers are combined and dried over MgSO₄ overnight. The solution is filtered and concentrated under reduced pressure. The residue is purified using a Kugelrohr (bulb to bulb) distillation.

Two of the required cyclic disulphides, 1,2-dithiane and 1,2-dithiacyclooctane, for Method C above are synthesised as follows.

1,2-Dithiane (Compound 28): Triethylamine (42.5 g), is dissolved in dichloromethane (400 ml) and cooled to 0°C in an ice bath. 1, 4-Butanedithiol (25 g) and iodine (51.9 g ) are added simultaneously so that the solution turns slightly yellow and the reaction temperature is below 5°C. After the end of the addition the reaction mixture is washed with dilute sodium thiosulphate solution (10%, 50 ml) and water (2 x 50 ml). The organic layer is separated and dried over magnesium sulphate overnight. The solid is filtered off and the solvent is evaporated (18 mbar, 50°C). The reaction gives 1,2-dithiane (24.6 g, 98% crude yield). The oil is dissolved in hexane (250 ml) and recrystallised (at -78°C) as a yellow solid (15.40 g, 63% isolated yield). The compound is unstable and is best stored in a brown jar in the dark and cold. Its characterising spectroscopic data are: δ_{H} 1.97 (4H, C*H*₂CH₂S), 2.85 (4H, C*H*₂S).

1,2-Dithiacyclooctane (Compound 29): Triethylamine (33.9 g) is dissolved in dichoromethane (400 ml) and cooled to 0°C in an ice bath. 1,6-Hexanedithiol (25 g) and iodine (42 g) are added simultaneously so that the solution turns slightly yellow and the reaction temperature is below 5°C. After the end of the addition the reaction mixture is washed with dilute sodium thiosulphate solution (10%, 50 ml) and water (2 x 50 ml). The organic layer is separated and dried over magnesium sulphate overnight. The solid is filtered off and the solvent is evaporated (18 mbar, 50°C). The reaction gives 1,2-dithiacyclooctane (88% crude yield). The oil is purified by Kugelrohr distillation (at 0.1 mbar). The fraction at 120°C is the pure compound, isolated in a yield of 68%. Its characterising spectroscopic data are δ_{H} 1.72 (4H, m, SCH₂CH₂C*H*₂), 1.93 (4H, m, SCH₂C*H*₂), 2.78 (4H, m, SC*H*₂); δ_{C} 24.52 (SCH₂CH₂*C*H₂), 25.52 (SCH₂*C*H₂), 37.97 (SCH₂); EI m/z (%), M⁺ = 148 (50), M⁺ = 55 (100), M⁺ = 41 (75).

Below are detailed the syntheses of Compounds 5 to 27 and compounds 30 to 38.

2,7-Dithiaoctane (Compound 5): The reaction is carried out as described in Method C using 1,2-dithiane (3.00 g, 25 mmol), methyllithium (18.0 ml, 25 mmol, 1.4 M) and iodomethane (3.55 g, 25 mmol) as reagents. The reaction gives 2,7-dithiaoctane (3.26 g, 85.9% crude yield). The Kugelrohr distillation is carried out at 0.1 mbar and the fraction (2.980 g) with a boiling point of 45°C is pure 2,7-dithiaoctane, isolated in a yield of 79.5%. Its characterising spectroscopic data are δ_{H} 1.71 (4H, m, SC*H*₂), 2.11 (6H, s , SC*H*₃), 2.52 (4H, m, SCH₂C*H*₂); δ_{C} 15.46 (*C*H₃), 28.01 (S*C*H₂), 33.76 (SCH₂*C*H₂). Elemental analysis found: C, 48.16; H, 9.53. C₆H₁₄S₂ requires C, 47.95; H, 9.39%.

2,8-Dithianonane (Compound 6): The reaction is carried out as described in Method A using 1,5-pentanedithiol (2.04 g, 15 mmol), n-butyllithium (12 ml, 30 mmol, 2.5 M) and iodomethane (4.26 g, 30 mmol) as reagents. The reaction gives 2,8-dithianonane (1.98 g, 79% crude yield). The Kugelrohr distillation is carried out at 0.2 mbar and the fraction (1.923 g) with a boiling point of 75°C is 2,8-dithianonane, isolated in a yield of 78%. Its m, characterising spectroscopic data are: δ_{H} 1.52 (2H, m, SCH₂CH₂C*H*₂), 1.62 (4H, m, SCH₂C*H*₂), 2.10 (6H, s, C*H*₃), 2.50 (4H, m, SC*H*₂); δ_{C} 15.52 (*C*H₃), 27.90 (SCH₂CH₂*C*H₂), 28.74 (SCH₂*C*H₂), 34.08 (S*C*H₂). Elemental analysis found: C, 51.08, H, 9.87. C₇H₁₆S₂ requires C, 51.17; H, 9.81%.

2,9-Dithiadecane (Compound 7): The reaction is carried out as described in Method C using DTCO (2.96 g, 20 mmol), methyllithium (14.3 ml, 20 mmol, 1.4 M, in diethyl ether) and iodomethane (2.84 g, 20 mmol) as reagents. The reaction gives 2,9-dithiadecane (2.37 g, 67% crude yield). The Kugelrohr distillation is carried out at 0.3 mbar and the fraction (1.963 g) with a boiling point of 90°C is pure 2,9-dithiadecane, isolated in a yield of 55%. Its characterising spectroscopic data are: δ_{H} 1.41 (4H, m, SCH₂CH₂C*H*₂), 1.61 (4H, m, SCH₂C*H*₂), 2.09 ( 6H, s, C*H*₃), 2.49 (4H, t, J7.4, SC*H*₂); δ_{C} 15.51 (*C*H₃), 28.36 (SCH₂CH₂CH₂CH₂), 28.99 (SCH₂*C*H₂), 34.17 (S*C*H₂). Elemental analysis found: C, 53.82; H, 10.38. C₈H₁₈S₂ requires C, 53.88; H, 10.17%.

2,10-Dithiaundecane (Compound 8): The reaction is carried out as described in Method B using methanethiol (2.00g, 40 mmol), n-butyllithium (16.7 ml, 42 mmol, 2.5 M) and 1,7-dibromoheptane (4.53 g, 17.6 mmol) as reagents. The reaction gives 2,10-dithiaundecane (3.33 g, 97% crude yield). The Kugelrohr distillation is carried out at 0.2 mbar and the fraction (3.117 g) with a boiling point of 60°C is pure 2,10-dithiaundecane, isolated in a yield of 93%. Its characterising spectroscopic data are: δ_{H} 1.36 ( 6H, m, C*H*₂), 1.62 (4H, m, SCH₂C*H*₂), 2.10 ( 6H, s, C*H*₃), 2.49 (4H, t J7.4, SC*H*₂); δ_{C} 15.53 (*C*H₃), 28.65, 28.83 (1/2), 29.06, 34.21 (*C*H₂). Elemental analysis found: C, 56.18; H, 10.23. C₉H₂₀S₂ requires C, 56.19; H, 10.48%.

2,11-Dithiadodecane (Compound 9): The reaction is carried out as described in Method A using 1,8-octanedithiol (2.0 g, 11.2 mmol), n-butyllithium (9.0 ml, 22.4 mmol, 2.5 M) and iodomethane (3.20 g, 22.4 mmol) as reagents. The reaction gives 2,11-dithiadodecane (1.44 g, 55% crude yield). The Kugelrohr distillation is carried out at 0.1 mbar and the fraction (1.176 g) with a boiling point of 100°C is pure 2,11-dithiadodecane, isolated in a yield of 45%. Its characterising spectroscopic data are: δ_{H} 1.34 (8H, m, SCH₂CH₂C*H*₂), 1.60 (4H, m, SCH₂C*H*₂), 2.10 ( 6H, s, 29.11 SC*H*₃), 2.49 (4H, t, J7.4, SC*H*₂), δ_{C} 15.53 (*C*H₃), 28.72, 29.11 (*C*H₂), 34.26 (S*C*H₂)

2,13-Dithiatetradecane (Compound 10): The reaction is carried out as described in Method Busing methanethiol (2.00 g, 40 mmol), n-butyllithium (16.7 ml, 40 mmol, 2.4 M) and 1,10-dibromodecane (6.00 g, 20 mmol) as reagents. The reaction gives 2,13-dithiatetradecane (4.69 g, 98% crude yield). The Kugelrohr distillation is carried out at 0.1 mbar and the fraction (4.557 g) with a boiling point of 120°C is pure 2,13-dithiatetradecane, isolated in a yield of 97%. Its characterising spectroscopic data are: δ_{H} 1.34 (12H, m, C*H*₂), 1.60 (4H, m, SCH₂C*H*₂), 2.10 (6H, s, C*H*₃), 2.49 (4H, t J7.4, SC*H*₂); δ_{C} 15.53 (CH₃), 28.79, 29.15, 29.21, 29.44 (*C*H₂), 34.72 (S*C*H₂). Elemental analysis found: C, 61.39; H, 11.12. C₁₂H₂₆S₂ requires C, 61.47; H, 11.18%.

2,15-Dithiahexadecane (Compound 11): The reaction is carried out as described in Method B using methanethiol (2.00 g, 40 mmol), n-butyllithium (16.7 ml, 40 mmol, 2.4 M) and 1,12-dibromododecane (6.56 g, 20 mmol) as reagents. The reaction gives 2,15-dithiahexadecane as a pure white solid (4.784 g, 91% isolated yield). Its characterising spectroscopic data are: δ_{H} 1.33 (16H, m, C*H*₂), 1.60 (4H, m, SCH₂C*H*₂), 2.10 (6H, s, C*H*₃), 2.49 (4H, t J7.42, SC*H*₂); δ_{C} 15.53 (*C*H₃), 28.81, 29.16, 29.24, 29.50, 29.55 (*C*H₂), 34.28 (S*C*H₂). Elemental analysis found: C, 63.77; H, 12.15. C₁₄H₃₀S₂ requires C, 64.08; H, 11.53%.

5,10-Dithiatetradecane (Compound 12): The reaction is carried out as described in Method C using 1,2-dithiane (1 g, 8.4 mmol), butyllithium (3.2 ml, 8.6 mmol, 2.7 M) and 1-bromobutane (1.6 g, 8.4 mmol) as reagents. The reaction gives 5,10-dithiatetradecane (1.70 g, 87% crude yield). The Kugelrohr distillation is carried out at 0.1 mbar and the fraction (1.620 g) with a boiling point of 235°C is pure 5,10-dithiatetradecane title compound, isolated in a yield of 83%. Its characterising spectroscopic data are: δ_{H} 0.92 (6H, t, J7.3, C*H*₃CH₂), 1.41 (4H, m CH₃C*H*₂CH₂), 1.56 (4H, m, C₂H₅C*H*₂CH₂), 1.69 (4H, m, SCH₂C*H*₂), 2.52 (8H, m, SCH₂); δ_{C} 13.65 (CH₃CH₂), 21.98 (CH₃CH₂), 28.70 (C₂H₅CH₂), 31. 64 (SCH₂CH₂), 31.74 (S*C*H₂), 31.76 (S*C*H₂); EI m/ z (%), 234 (M⁺, 15), 177 (100), 145 (42), 121 (50); CI m/z (%), 235 ([M⁺ + 1], 52), 177 (20), 145 (100).

5,11-Dithiapentadecane (Compound 13): The reaction is carried out as described in Method A using 1,5-pentanedithiol (2.73 g, 20 mmol), n-butyllithium (16.0 ml, 40 mmol, 2.5 M) and 1-bromobutane (5.50 g, 40 mmol) as reagents. The reaction gives 5,11-dithiapentadecane (4.02 g, 76% crude yield). The Kugelrohr distillation is carried out at 0.1 mbar and the fraction (3.607 g) with a boiling point of 120°C is pure 5,11-dithiapentadecane, isolated in a yield of 73%. Its characterising spectroscopic data are: δ_{H} 0.92 (6H, t, J7.3, C*H*₃), 1.51 (14H, m, C*H*₂), 2.51 (8H, m, SC*H*₂); δ_{C} 13.70 (*C*H₃), 22.02, 28.18 (½), 29.30, 31.77 (*C*H₂), 31.82, 31.95 (S*C*H₂). Elemental analysis found: C, 62.54; H, 11.68. C₁₃H₂₈S₂ requires C, 62.84; H, 11.36%.

5,13-Dithiaheptadecane (Compound 15): The reaction is carried out as described in Method B using 1-butanethiol (3.61 g, 40 mmol), n-butyllithium (16 ml, 40 mmol, 2.5 M) and 1,7-dibromoheptane (5.16 g, 20 mmol) as reagents. The reaction gives 5,13-dithiaheptadecane (5.08 g, 90% crude yield). The Kugelrohr distillation is carried out at 0.1 mbar and the fraction (4.850 g) with a boiling point of 120°C is pure 5,13-dithiaheptadecane, isolated in a yield of 88%. Its characterising spectroscopic data are: δ_{H} 0.92 (6H, t, J7.3, C*H*₃), 1.38 (10H, m, SCH₂CH₂C*H*₂ and SCH₂CH₂CH₂C*H*₂), 1.58 (8H, m, SCH₂C*H*₂), 2.50 (8H, m, SC*H*₂); δ_{C} 13.73 (*C*H₃), 28.81, 28.88, 29.63 (*C*H₂), 31.82, 31.85, 32.12 (S*C*H₂). Elemental analysis found: C, 65.25; H, 11.82. C₁₅H₃₂S₂ requires C, 65.15; H, 11.66%.

5,14-Dithiaoctadecane (Compound 16): The reaction is carried out as described in Method A using 1,8-octanedithiol (2.0 g, 11.2 mmol), n-butyllithium (9 ml, 22 mmol, 2.5 M) and 1-bromobutane (3.07 g, 22.4 mmol) as reagents. The reaction gives 5,14-dithiaoctadecane (2.91 g, 86% crude yield). The Kugelrohr distillation is carried out at 0.1 mbar and the fraction (2.523 g) with a boiling point of 140°C is pure 5,14-dithiaoctadecane, isolated in a yield of 78%. Its characterising spectroscopic data are: δ_{H} 0.92 ( 6H, t, J7.3, C*H*₃), 1.37 (12H, m, C*H*₂), 1.58 (8H, m, SCH₂C*H*₂), 2.50 (8H, m, SC*H*₂), δ_{C} 13.71 (*C*H₃), 22.04, 28.86, 29.12, 29.66 (*C*H₂), 31.81 (S*C*H₂CH₂), 32.12 (S*C*H₂).

5,16-Dithiaeicosane (Compound 17): The reaction is carried out as described in Method B using 1-butanethiol (3.61 g, 40 mmol), n-butyllithium (16.0 ml, 40 mmol, 2.5 M) and 1,10-dibromodecane (6.00 g, 20 mmol) as reagents. The reaction gives 5,16-dithiaeicosane(6.11 g, 95% crude yield). The Kugelrohr distillation is carried out at 0.1 mbar and the fraction (5.894 g) with a boiling point of 150°C is pure 5,16-dithiaeicosane, isolated in a yield of 93%. Its characterising spectroscopic data are: δ_{H} 0.92 ( 6H, t, J7.3, C*H*₃), 1. 28 (8H, m, C*H*₂), 1. 39 (8H, m, SCH₂CH₂CH₂), 1.57 (8H, m, SCH₂C*H*₂), 2.50 (8H, m, SC*H*₂); δ_{C} 13.74, 22.07 (*C*H₂), 28.95-29.72 (*C*H₂), 31.84 (S*C*H₂), 32.17 (S*C*H₂). Elemental analysis found: C. 67.61; H, 12.07. C₁₈H₃₈S₂ requires C, 67.85; H, 12.02%.

5,18-Dithiadocosane (Compound 18): The reaction is carried out as described in Method B using 1-butanethiol (3.61 g, 40 mmol), n-butyllithium (16 ml, 40 mmol, 2.5 M) and 1,12-dibromododecane (6.56 g, 20 mmol) as reagents. The reaction gives 5,18-dithiadocosane as a pure white solid (6.70 g), isolated in a yield of 97%. Its characterising spectroscopic data are: δ_{H} 0.92 (6H, t, J7.33, C*H*₃), 1.26 (12H, m, SCH₂C*H*₂CH₂CH₂), 1.39 (8H, m, SCH₂CH₂C*H*₂), 1.58 (8H, m, SCH₂C*H*₂), 2 . 50 (8H, m, SC*H*₂); δ_{C} 13.71 (*C*H₃), 22.04 (SCH₂CH₂*C*H₂CH₃), 28.94, 29.24, 29.41, 29.51, 29.55, 29.71 (*C*H₂), 31.81 (S*C*H₂). Elemental analysis found: C, 69.15; H, 12.01. C₂₀H₄₂S₂ requires C, 69.29; H, 12.21%.

4,11-Dithiatetradecane (Compound 19): The reaction is carried out as described in Method A using 1,6-hexanedithiol (3.00 g, 20 mmol), n-butyllithium (16.0 ml, 40 mmol, 2.5 M) and 1-bromopropane as reagents. The reaction gives 4,11-dithiatetradecane (4.47 g, crude yield. The Kugelrohr distillation is carried out at 0.05 mbar and the fraction (4.322 g) with a boiling point of 125°C is pure 4,11-dithiatetradecane, isolated in a yield of 92%. Its characterising spectroscopic data are: δ_{H} 0.99 (6H, t, J7.35, C*H*₃), 1.40 (4H, m, C*H*₂), 1.61 (8H, m, SCH₂C*H*₂), 2.49 (8H, m, SC*H*₂); δ_{C} 13.55 (*C*H₃), 23.01 (*C*H₂CH₃), 28.54, 29.59 (*C*H₂), 32.02 (S*C*H₂), 34.21 (S*C*H₂). Elemental analysis found: C, 61.22; H, 11.22. C₁₂H₂₆S₂ requires C, 61.47; H, 11.18%.

5,12-Dithiahexadecane (Compound 20): The reaction is carried out as described in Method C using DTCO (2.09 g, 14.1 mmol), n-butyllithium (1.5 ml, 14.1 mmol, 9.6 M) and 1-bromobutane (1.9 g, 14.1 mmol) as reagents. The reaction gives 5,12-dithiahexadecane (2.98 g, 80% crude yield). The Kugelrohr distillation is carried out at 0.05 mbar and the fraction (2.571 g) with a boiling point of 120°C is pure 5,12-dithiahexadecane, isolated in a yield of 70%. Its characterising spectroscopic data are: δ_{H} 0.94 (6H, t, J7.3, CH₃), 1.41 (8H, m, SCH₂CH₂C*H*₂), 1.59 (8H, m, SCH₂C*H*₂), 2.50 (8H, t, J7.4, SC*H*₂); δ_{C} 13.69 (*C*H₃). 22.02 (CH₃*C*H₂), 28.51 (SCH₂CH₂*C*H₂), 29.54 (SCH₂*C*H₂), 31.79 (CH₃CH₂*C*H₂), 31.82 (S*C*H₂), 32.05 (CH₃CH₂CH₂*C*H₂S); EI m/z (%), 262 (M⁺, 10), 173 (35), 115 (50), 82 (45), 61 (100), 55 (65), 41 (50); CI m/z (%), 263 ([M⁺ + 1], 100), 173 (15).

6,13-Dithiaoctadecane (Compound 21): The reaction is carried out as described in Method A using 1,6-hexanedithiol (3.00 g, 20 mmol), n-butyllithium (16.0 ml, 40 mmol, 2.5 M) and 1-bromopentane (6.04 g, 40 mmol) as reagents. The reaction gives 6, 13-dithiaoctadecane (5.55 g, 92% crude yield). The Kugelrohr distillation is carried out at 0.1 mbar and the fraction (5.230 g) with a boiling point of 150°C is pure 6,13-dithiaoctadecane, isolated in a yield of 90%. The characterising spectroscopic data are: δ_{H} 0.90 (6H, t, m, C*H*₃), 1.36 (12H, m, C*H*₂), 1.58 (8H, m, SCH₂C*H*₂), 2.50 (8H, m, SC*H*₂); δ_{C} 13.40 (*C*H₃), 22.32, 28.53, 29.40, 29.56, 31.13 (*C*H₂), 32.06, 32.14 (S*C*H₂). Elemental analysis found: C, 66.43; H, 11.52. C₁₆H₃₄S₂ requires C, 66.14; H, 11.79%.

8,15-Dithiadocosane (Compound 22): The reaction is carried out as described in Method A using 1,6-hexanedithiol (1.05 g, 7 mmol), n-butyllithium (5.6 ml, 14 mmol, 2.5 M) and 1-iodoheptane (3.165 g, 14 mmol) as reagents. The reaction gives 8,15-dithiadocosane (2.37 g, 96% crude yield). The Kugelrohr distillation is carried out at 0.2 mbar and the fraction (2.129 g) with a boiling point of 175°C is pure 8,15-dithiadocosane title compound, isolated in a yield of 88%. Its characterising spectroscopic data are: δ_{H} 0.88 ( 6H, t J6.9, C*H*₃), 1.33 (20H, m, C*H*₂), 1.57 (8H, m, SCH₂C*H*₂), 2.50 (8H, m, SC*H*₂); δ_{C} 14.07 (*C*H₃), 22.60, 28.52, 28.91, 29.56, 29.71, 31.74 (*C*H₂), 32.06, 32.17 (S*C*H₂). Elemental analysis found: C, 69.31; H, 12.21. C₂₀H₄₂S₂ requires C, 69.29; H, 12.21%.

3,16-Dithiaoctadecane (Compound 23): The reaction is carried out as described in Method B using ethanethiol (1.24 g, 20 mmol), n-butyllithium (8.3 ml, 20 mmol, 2.4M) and 1,12-dibromododecane (3.28 g, 10 mmol) as reagents. The reaction gives 3,16-dithiaoctadecane (2.92 g, 94% crude yield). The Kugelrohr distillation is carried out at 0.2 mbar and the fraction (2.554 g) with a boiling point of 155°C is pure 3,16-dithiaoctadecane, isolated in a yield of 88%. Its characterising spectroscopic data are: δ_{H} 1.30 ( 22H, m, C*H*₂ and C*H*₃), 1.59 (4H, m, SCH₂C*H*₂), 2.53 (8H, m, SC*H*₂); δ_{C} 14.80 (*C*H₃), 25.89, 28.94, 29.24, 29.50, 29.54, 29.63 (*C*H₂), 31.94 (S*C*H₂). Elemental analysis found: C, 66.68; H, 11.66. C₁₆H₃₄S₂ requires C, 66.14; H, 11.79%.

4,17-Dithiaeicosane (Compound 24): The reaction is carried out as described in Method B using 1-propanethiol (1.52 g, 20 mmol), n-butyllithium (8.3 ml, 20 mmol, 2.4 M) and 1,12-dibromododecane (3.28 g, 10 mmol) as reagents. The reaction gives 4,17-dithiaeicosane (3.20 g, 93% crude yield). The Kugelrohr distillation is carried out at 0.2 mbar and the fraction (2.708 g) with a boiling point of 180°C is the pure title compound, isolated in a yield of 85%. Its characterising spectroscopic data are: δ_{H} 0.92 ( 6H, t, J7.3, C*H*₃), 1.32 (16H, m, C*H*₂), 1.58 (8H, m, SCH₂C*H*₂), 2.49 (8H, m, SC*H*₂); δ_{c} 13.57 (*C*H₃), 23.02, 28.98, 29.27, 29.54, 29.59, 29.76, 32.12 (*C*H₂), 34.23 (S*C*H₂). Elemental analysis found: C, 67.97; H, 11.93. C₁₈H₃₈S₂ requires C, 67.85; H, 12.02%.

6,19-Dithiatetracosane (Compound 25): The reaction is carried out as described in Method B using 1-pentanethiol (2.08 g, 20 mmol), n-butyllithium (8.0 ml, 20 mmol, 2.5 M) and 1,12-dibromododecane (3.28 g, 10 mmol) as reagents. The reaction gives 6,19-dithiatetracosane(3.75 g, 95% crude yield). The Kugelrohr distillation is carried out at 0.2 mbar and the fraction (3.155 g) with a boiling point of 160°C is pure 6,19-dithiatetracosane, isolated in a yield of 84%. Its characterising spectroscopic data are: δ_{H} 0.90 ( 6H, t J7.1, C*H*₃), 1.30 (24H, m, C*H*₂), 1.58 (8H, m, SCH₂C*H*₂), 2.50 (8H, t J7.1, SC*H*₂); δ_{C} 13.98 (*C*H₃), 22.32, 28.94, 29.25, 29.40, 29.51, 29.56, 29.71, 31.12 (*C*H₂), 32.12, 32.15 (S*C*H₂). Elemental analysis found: C, 70.60; H, 13.42. C₂₂H₄₆S₂ requires C, 70.52; H, 12.37%.

7,20-Dithiahexacosane (Compound 26): The reaction is carried out as described in Method B using 1-hexanethiol (2.43 g, 20 mmol), n-butyllithium (8.0 ml, 20 mmol, 2.5 M) and 1,12-dibromododecane (3.28 g, 10 mmol) as reagents. The reaction gives 7,20-dithiahexacosane (3.944 g, 91% crude yield). The Kugelrohr distillation is carried out at 0.2 mbar and the fraction (3.298 g) with a boiling point of 235°C is pure 7,20-dithiahexacosane, isolated in a yield of 82%. Its characterising spectroscopic data are: δ_{H} 0.89 (6H, t, J6.9, C*H*₃), 1.33 (28H, m, C*H*₂), 1.57 (8H, m, SCH₂C*H*₂), 2.50 (8H, m, SC*H*₂); δ_{C} 14.06 (*C*H₃), 22.58, 28.66, 28.97, 29.27, 29.53, 29.70, 29.72, 31.48 (*C*H₂), 32.17 (S*C*H₂). Elemental analysis found: C, 71.44; H, 13.73. C₂₄H₃₀S₂ requires C, 71.57; H, 12.51%.

8,21-Dithiaoctacosane (Compound 27): The reaction is carried out as described in Method B using 1-heptanethiol (2.73 g, 20 mmol), n-butyllithium (8.0 ml, 20 mmol, 2.5 M) and 1,12-dibromododecane (3.28 g, 10 mmol) as reagents. The reaction gives 8,21-dithiaoctacosane(4.34 g, 99% crude yield). The Kugelrohr distillation is carried out at 0.15 mbar and the fraction (3.641 g) with a boiling point of 255°C is pure 8,21-dithiaoctacosane, isolated in a yield of 85%. Its characterising spectroscopic data are: δ_{H} 0.88 ( 6H, m, C*H*₃), 1.32 (32H, m, C*H*₂), 1.57 (8H, m, SCH₂C*H*₂), 2.50 (8H, m, SC*H*₂); δ_{C} 14.07 (*C*H₃), 22.61, 28.92, 28.94, 29.26, 29.36, 29.51, 29.57, 29.65, 29.72, 31.74, 32.16 (*C*H₂). Elemental analysis found: C, 72.57; H, 14.13. C₂₆H₅₄S₂ requires C, 72.48; H, 12.63%.

2,7-Dithiaundecane (Compound 30): The reaction is carried out as described in Method C using 1,2-dithiane (3.00 g, 25 mmol), methyllithium (18 ml, 25 mmol, 1.4 M, in diethyl ether) and 1-bromobutane (3.43 g, 25 mmol) as reagents. The reaction gives 2,7-dithiaundecane (4.61 g, 81% crude yield). The Kugelrohr distillation is carried out at 0.1 mbar and the fraction (3.502 g) with a boiling point of 100°C is pure 2,7-dithiaundecane, isolated in a yield of 73%. Its characterising spectroscopic data are: δ_{H} 0.92 (3H, t, J7.3, C*H*₃CH₂), 1.41 (2H, m, CH₃C*H*₂*C*H₂), 1.57 (2H, m, C₂H₅C*H*₂), 1 . 70 (4H, m, SCH₂C*H*₂), 2.10 (3H, s, SC*H*₃), 2.52 (6H, m, SC*H*₂); δ_{C} 13.72 (*C*H₃CH₂), 15.48 (S*C*H₃), 22.03 (CH₃*C*H₂), 28.04 (SCH₂*C*H₂), 28.16 (SCH₂*C*H₂), 28.60 (SCH₂*C*H₂), 31.65 (S*C*H₂), 33,79 (CH₃S*C*H₂); El m/z (%), 192 (M⁺, 30), 135 (95), 103 (55), 87 (50), 61 (100); Cl m/z (%), 210 ([M⁺ + 1 + NH₃], 10), 193 ([M⁺ + 1], 100), 145 (50), 103 (100).

Elemental analysis found: C, 55.98; H, 11.49. C₉H₂₀S₂ requires C, 56.19; H, 10.48%.

3,8-Dithiadodecane (Compound 31): The reaction is carried out as described in Method C using 1,2-dithiane (1.00 g, 8.4 mmol), n-butyllithium (3.2 ml, 8.4 mmol, 2.7 M) and bromoethane (0.92 g, 8.4 mmol) as reagents. The reaction gives 3,8-dithiadodecane (1.85 g, 97% crude yield). The Kugelrohr distillation is carried out at 0.05 mbar and the fraction (1.320 g) with a boiling point of 100°C is pure 3,8-dithiadodecane, isolated in a yield of 77%. Its characterising spectroscopic data are: δ_{H} 0.92 (3H, t, J7.3, C₂H₅C*H*₃), 1.26 (3H, t, J7.4, C*H*₃CH₂), 1.41 (2H, m, CH₃C*H*₂), 1.56 ( 2H, m, SCH₂C*H*₂C₂H₅), 1. 69 (4H, m, SCH₂C*H*₂), 2.53 (8H, m, SC*H*₂); δ_{C} 13.68 (*C*H₃C₂H₅), 14.76 (SCH₂*C*H₃), 21.99 (CH₃*C*H₂), 25.83 (C₂H₅*C*H₂), 28. 69 (SCH₂*C*H₂CH₂), 28.90 (SCH₂*C*H₂CH₂), 31.13(S*C*H₂CH₂), 31.62 (S*C*H₂CH₂), 31.73 (S*C*H₂CH₂); EI m/z (%), 206 (M⁺, 20), 177 (85), 149 (85), 121 (100), 87 (85); CI m/z (%), 207 ([M⁺ + 1], 100), 145 (80), 117 (100).

2,2-Dimethyl-3,8-dithiadodecane (Compound 32): The reaction is carried out as described in Method C using 1,2-dithiane (1.00 g, 8.4 mmol), tert-butyllithium (1.5 ml, 8.4 mmol, 5.6 M, in pentane) and 1-bromobutane (1.2 g, 8.4 mmol) as reagents. The reaction gives 2,2-dimethyl-3,8-dithiadodecane (1.93 g, 98% crude yield). The Kugelrohr distillation is carried out at 0.05 mbar and the fraction (1.430 g) with a boiling point of 100°C is pure 2,2-dimethyl-3,8-dithiadodecane, isolated in a yield of 75%. Its characterising spectroscopic data are: δ_{H} 0.92 (3H, t, J7.3, CH₂C*H*₃), 1.32 (9H, s, C(C*H*₃)₃), 1.42 (2H, m, CH₃C*H*₂), 1.56 (2H, m, C₂H₅C*H*₂), 1.69 (4H, m, SCH₂C*H*₂), 2.53 (6H, m, SC*H*₂); δ_{C} 13.69 (*C*H₃CH₂), 21.99 (CH₃*C*H₂), 27.82(C₂H₅*C*H₂), 28.95 (SCH₂*C*H₂CH₂), 29.07 (SCH₂*C*H₂CH₂), 30.95 (C(*C*H₃)₃), 31.62 (CH₂S*C*H₂), 31.74 (S*C*H₂), 41.83 (*C*(CH₃)₃); EI m/z (%), 234 (M⁺, 51), 177 (100), 121 (85), 87 (50), 57 (55), 41 (35); CI m/z (%), 235 ([M⁺ + 1], 95), 177 (30), 145 (100).

1-Phenyl-2,7-dithiaundecane (Compound 33): The reaction is carried out as described in Method C using 1,2-dithiane (1.00 g, 8.4 mmol), n-butyllithium (3.2 ml, 8.4 mmol, 2.7 M) and benzyl bromide (1.44g, 8.4 mmol) as reagents. The reaction gives 1-phenyl-2,7-dithiaundecane(2.24 g, 79% crude yield). The Kugelrohr distillation is carried out at 0.1 mbar and the fraction (1.475 g) with a boiling point of 235°C is pure 1-phenyl-2,7-dithiaundecane, isolated in a yield of 66.0%. Its characterising spectroscopic data are: δ_{H} 0.91 (3H, t, J7.3, CH₂C*H*₃), 1.41 (2H, m, C*H*₂CH₃), 1.59 ( 6H, m, SCH₂C*H*₂), 2.46 ( 6H, m, SC*H*₂), 3.69 ( 2H, s, PhC*H*₂S), 7.23 (1H, m, *p*-Ph), 7.30 (4H, m, Ph), δ_{C} 13.71 (CH₂*C*H₃), 22.02 (*C*H₂CH₃), 28.28 (*C*H₂CH₂CH₃), 28.64 (SCH₂*C*H₂), 30.83 (S*C*H₂C₂H₅), 31.66 (S*C*H₂), 31.78 (S*C*H₂), 36.20 (S*C*H₂Ph), 126.89 (*para*-Ph), 128.44 (ortho-Ph), 128.82 (*meta*-Ph), 138.52 (*ipso-*Ph); EI m/z (%), 214 (2), 177 (75), 121 (45), 91 (100); CI m/z (%), 269 ([M⁺ + 1], 85), 179 (100), 145 (90).

1-Phenyl-1,6-dithiadecane (Compound 34): The reaction is carried out as described in Method C using 1,2-dithiane (1.00 g, 8.4 mmol), phenyllithium (5.6 ml, 8.4 mmol, 1.5 M, in cyclohexane-diethyl ether 70/30)) and 1-bromobutane (1.6 g, 8.4 mmol) as reagents. The reaction gives 1-phenyl-1,6-dithiadecane (1.83 g, 77% crude yield). The Kugelrohr distillation is carried out at 0.4 mbar and the fraction (1.320 g) with a boiling point of 120-150°C is pure 1-phenyl-1,6-dithiadecane, isolated in a yield of 62%. Its characterising spectroscopic data are: δ_{H} 0.91 (3H, t, J7.3, C*H*₃CH₂), 1.39 (2H, m, CH₃C*H*₂), 1.55 (2H, m, C₂H₅C*H*₂), 1.73 (4H, m, SCH₂C*H*₂), 2.50 (4H, m, SC*H*₂), 2.93 (2H, m, PhSC*H*₂), 7.16 (1H, m, *p*-PhS), 7.29 (4H, m, PhS); δ_{c} 13.73 (*C*H₃), 22.04 (CH₃*C*H₂), 28.23 (C₂H₅*C*H₂), 28.62 (SCH₂*C*H₂), 28.74 (SCH₂*C*H₂), 31.56 (S*C*H₂), 31.78 (S*C*H₂), 33.23 (PhS*C*H₂), 125.82 (*para*-PhS), 128.86 (meta-PhS), 129.06 (ortho-PhS), 136.63 (*ipso*-PhS); EI m/z (%), 254 (M⁺, 40), 197 (45), 145 (100), 123 (45), 89 (75), 61 (55), 55 (60), 41 (50); CI m/z (%), 255 ([M⁺ + 1], 60), 272 (M⁺ + NH₃, small), 165 (95), 145 (100).

1-Phenyl-8-methyl-1,6-dithianonane (Compound 35): The reaction is carried out as described in Method C using 1,2-dithiane (2.02 g, 15 mmol), phenyllithium (8.8 ml, 16 mmol, 1.8 M, in cyclohexane-diethyl ether 70/30) and 1-bromo-2-methyl propane (2.06 g, 15 mmol) as reagents. The reaction gives 1-phenyl-8-methyl-1,6-dithianonane (3.35 g, 76% crude yield). The Kugelrohr distillation is carried out at 0.1 mbar and the fraction (2.716 g) with a boiling point of 175°C is pure 1-phenyl-8-methyl-1,6-dithianonane, isolated in a yield of 71%. Its characterising spectroscopic data are: δ_{H} 0.97 ( 6H, d, J6.6, C*H*₃), 1.75 (5H, m, SCH₂C*H*₂ and CH), 2.38 (2H, d, J6.9, SC*H*₂CH (CH₃)₂), 2.50 (2H, t, J7.0, SC*H*₂CH₂), 2.93 (2H, t, J6.9, PhSC*H*₂), 7.16 (1H, m, *p*-Ph), 7 . 29 (4H, m, *Ph*); δ_{C} 22.05 (*C*H₃), 28.19 (SCH₂*C*H₂), 28.57 (SCH₂*C*H₂), 28.66 (CH), 32.17 (S*C*H₂), 33.18 (S*C*H₂), 44.42 (PhS*C*H₂), 125.79 (*para*-Ph), 128.83 (Ph), 129.03 (Ph), 136.58 (*ipso-*Ph). Elemental analysis found:C, 65.94; H, 8.70. C₁₄H₂₂S₂ requires C, 66.09; H, 8.71%.

1,7-Diphenyl-1,6-dithiaheptane (Compound 36): The reaction is carried out as described in Method C using 1,2-dithiane (1.00 g, 8.4 mmol), phenyllithium (5.6 ml, 8.4 mmol, 1.5 M, in cyclohexane-diethyl ether 70/30) and benzyl bromide (1.4 g, 8.4 mmol) as reagents. The reaction gives 1,7-diphenyl-1,6-dithiaheptane (2.4 g, 99% crude yield). The Kugelrohr distillation is carried out at 0.05 mbar and the fraction (2.250 g) with a boiling point of 190-200°C is pure 1,7-diphenyl-1,6-dithiaheptane, isolated in a yield of 94%. Its characterising spectroscopic data are: δ_{H} 1.68 (4H, m, SCH₂C*H*₂), 2.39 (2H, m, CH₂SC*H*₂), 2.87 (2H, m, PhS*CH*₂), 3.67 (2H, s, SC*H*₂Ph), 7.16 (1H, m, *para*-PhS), 7.27 (9H, m, Ph); δ_{*c*} (¹H¹³C correlation spectra) 28.11 (SCH₂*C*H₂, 2x), 30.70 (CH₂S*C*H₂); 30.77 (PhS*C*H₂), 33.15 (Ph*C*H₂S), 125.86 (*para*-Ph), 126.94 (*para*-PhS), 128.50 - 129.21 (Ph), 136.60 (ipso-Ph), 138.46 (ipso-PhS); EI m/z (%), 211 (5), 197 ([M⁺- CH₂-Ph], 95), 91 (100); CI m/z (%), 289 (M⁺+1, 40), 197 (35), 179 (100), 165 (85).

5,12-Dithianonadecane (Compound 37): The reaction is carried out as described in Method C using DTCO (1.24 g, 8.4 mmol), n-butyllithium (3.2 ml, 8.4 mmol), 2.7 M) and 1-iodoheptane (1.9 g, 8.4 mmol) as reagents. The reaction gives 5,12-dithianonadecane (2.261 g, 82% crude yield). The Kugelrohr distillation is carried out at 0.1 mbar and the fraction (1.557 g) with a boiling point of 170-180°C is pure 5,12-dithianonadecane, isolated in a yield of 61%. Its characterising spectroscopic data are: δ_{H} 0.90 (6H, m, C*H*₃), 1.30 (6H, m, CH₃C*H*₂C*H*₂C*H*₂CH₂CH₂), 1.41 (8H, m, SCH₂CH₂C*H*₂), 1.56 (8H, m, SCH₂C*H*₂), 2.50 (8H, m, SC*H*₂); δ_{C} (¹H¹³C correlation spectra) 13.71 (*C*H₃), 14.09 (*C*H₃), 22.05 (SCH₂CH₂*C*H₂), 22.62 (CH₃*C*H₂), 28.54 (SCH₂CH₂*C*H₂), 28.93 (CH₃CH₂*C*H₂ and SCH₂CH₂*C*H₂), 29.58 (SCH₂*C*H₂), 29.73 (SCH₂*C*H₂), 29.73 (SCH₂*C*H₂), 31.76 (CH₃CH₂CH₂*C*H₂), 31.82 (SCH₂*C*H₂), 31.85 (S*C*H₂), 32.08 (S*C*H₂); 32.20 (S*C*H₂); EI m/z (%), 346 (5, C₇H₁₅-S-C₆H₁₂-S-C₇H₁₅ as a minor impurity), 304 (M⁺, 15), 215 (65), 115 (100), 82 (70), 55 (95); CI m/z (%), 347 (see above, 30), 305 ([M⁺ + H], 100), 263 (20).

1-Phenyl-2,9-dithiatridecane (Compound 38): The reaction is carried out as described in Method C using DTCO (1.24g, 8.4 mmol), n-butyllithium (3.2 ml, 8.4 mmol, 2.7 M) and benzyl bromide (1.4 g, 8.4 mmol) as reagents. The reaction gives 1-phenyl-2,9-dithiatridecane (2.19 g, 86% crude yield). The Kugelrohr distillation is carried out at 0.01 mbar and the fraction (1.117 g) with a boiling point of 175°C is pure 1-phenyl-2,9-dithiatridecane, isolated in a yield of 62%. Its characterising spectroscopic data are: δ_{H} 0.91 (3H, t, J7.3, C*H*₃), 1.39 (6H, m, SCH₂CH₂C*H*₂), 1.56 (6H, m, SCH₂C*H*₂), 2.40 (2H, t, J7.4, C*H*₂SCH₂Ph), 2.49 (4H, m, SC*H*₂), 3.69 (2H, s, SC*H*₂Ph), 7.24 (1H, m, p-Ph), 7.30 (4H, m, *Ph*); δ_{C} 13.73 (*C*H₃), 22.05 (CH₃*C*H₂), 28.45 (SCH₂CH₂*C*H₂), 28.48 (SCH₂CH₂*C*H₂), 29.06 (CH₃CH₂*C*H₂CH₂S), 30.21 (SCH₂*C*H₂), 31.27 (SCH₂*C*H₂), 31.82 (S*C*H₂), 31.85 (S*C*H₂), 32.05 (S*C*H₂), 36.29 (S*C*H₂Ph), 126.86 (*para*-Ph), 128.44 (*ortho*-Ph), 128.82 (*meta*-Ph), 138.62 (ipso-Ph). Elemental analysis found: C, 69.17; H, 10.04. C₂₆H₅₄S₂ requires C, 68.90; H, 9.50%.

Tables J, K and L below summarise the above syntheses, together with some additional results in Table J, namely preparation of Compound No. 12 by method B and preparation of Compound No. 14.

**Table J**

| compound number | method^{a} | product | isolated yield % |
|---|---|---|---|
| 5 | C | C₁-S-C₄-S-C₁ | 80 |
| 6 | A | C₁-S-C₅-S-C₁ | 78 |
| 7 | C | C₁-S-C₆-S-C₁ | 55 |
| 8 | B | C₁-S-C₇-S-C₁ | 93 |
| 9 | A | C₁-S-C₈-S-C₁ | 45 |
| 10 | B | C₁-S-C₁₀-S-C₁ | 97 |
| 11 | B | C₁-S-C₁₂-S-C₁ | 91 |
| 12 | B/C | C₄-S-C₄-S-C₄ | 94/83 |
| 13 | B | C₄-S-C₅-S-C₄ | 73 |
| 14 | C | C₄-S-C₆-S-C₄ | 70 |
| 15 | B | C₄-S-C₇-S-C₄ | 88 |
| 16 | A | C₄-S-C₈-S-C₄ | 78 |
| 17 | B | C₄-S-C₁₀-S-C₄ | 93 |
| 18 | B | C₄-S-C₁₂-S-C₄ | 97 |

**Table K**

| compound number | method^{a} | product | isolated yield % |
|---|---|---|---|
| 19 | A | C₃-S-C₆-S-C₃ | 92 |
| 20 | C | C₄-S-C₆-S-C₄ | 70 |
| 21 | A | C₅-S-C₆-S-C₅ | 90 |
| 22 | A | C₇-S-C₆-S-C₇ | 88 |
| 23 | B | C₂-S-C₁₂-S-C₂ | 82 |
| 24 | B | C₃-S-C₁₂-S-C₃ | 85 |
| 25 | B | C₅-S-C₁₂-S-C₅ | 84 |
| 26 | B | C₆-S-C₁₂-S-C₆ | 82 |
| 27 | B | C₇-S-C₁₂-S-C₇ | 85 |

**Table L**

| comp. number | RLi | disulfide | EX | product | crude yield % |
|---|---|---|---|---|---|
| 5 | MeLi | 1,2-Dithiane | Mel | C₁-S-C₄-S-C₁ | 86 |
| 30 | MeLi | " | BuBr | C₁-S-C₄-S-C₄ | 81 |
| 31 | BuLi | " | EtBr | C₄-S-C₄-S-C₂ | 97 |
| 12 | BuLi | " | BuBr | C₄-S-C₄-S-C₄ | 87 |
| 32 | Bu^{tert}Li | " | BuBr | C₄^{tert}-S-C₄-S-C₄ | 98 |
| 33 | BuⁿLi | " | PhCH₂Br | C₄-S-C₄-S-CH₂Ph | 78 |
| 34 | PhLi | " | BuBr | Ph-S-C₄-S-C₄ | 77 |
| 35 | PhLi | " | Bu^{iso}Br | Ph-S-C₄-S-C₃^{iso} | 76 |
| 36 | PhLi | " | PhCH₂Br | Ph-S-C₄-S-CH₂Ph | 98 |
| 7 | MeLi | DTCO | Mel | C₁-S-C₆-S-C₁ | 67 |
| 20 | BuLi | " | BuBr | C₄-S-C₆-S-C₄ | 85 |
| 37 | BuLi | " | C₇H₁₅Br | C₄-S-C₆-S-C₇ | 82 |
| 38 | BuLi | " | PhCH₂Br | C₄-S-C₆-S-CH₂Ph | 86 |

### Examples 1 to 4

### Example 1

Table 1 below illustrates the effectiveness of various catalysts of general formula (II), with both R³ and R⁴ being methyl groups, each R⁵ having a different chain length, in both the absence and presence of AlCl₃. The para:ortho ratios increase with the length of the R⁵ group, and the best results are achieved with C₁₀ to C₁₂.

**Table 1**

| Chlorination of *meta*-cresol in the presence of dithiaalkanes^{a} | | | | | | |
|---|---|---|---|---|---|---|
| catalyst | presence of AlCl₃ | MC mol% | OCMC mol% ^{b} | PCMC mol%^{b} | *p*/*o* ratio | mass balance %^{c} |
| C₁-S-C₄-S-C₁ | - | 6.2 | 15.2 | 70.6 | 4.6 | 92.0 |
| C₁-S-C₄-S-C₁ | √ | 13.4 | 6.8 | 54.8 | 8.1 | 75.0 |
| C₁-S-C₅-S-C₁ | - | 3.6 | 10.8 | 82.4 | 7.6 | 96.8 |
| C₁-S-C₅-S-C₁ | √ | 0.4 | 9.0 | 82.8 | 9.2 | 92.2 |
| C₁-S-C₆-S-C₁ | - | 7.6 | 10.2 | 77.8 | 7.6 | 95.6 |
| C₁-S-C₆-S-C₁ | √ | 15.8 | 7.6 | 69.8 | 9.2 | 93.2 |
| C₁-S-C₇-S-C₁ | - | 14.0 | 6.8 | 67.2 | 9.9 | 88.0 |
| C₁-S-C₇-S-C₁ | √ | 1.3 | 5.6 | 93.1 | 16.6 | 100.0 |
| C₁-S-C₈-S-C₁ | - | 9.6 | 9.2 | 66.8 | 7.3 | 85.6 |
| C₁-S-C₈-S-C₁ | √ | 14.2 | 6.6 | 74.8 | 11.3 | 95.6 |
| C₁-S-C₁₀-S-C₁ | - | 5.0 | 7.8 | 82.9 | 10.6 | 95.7 |
| C₁-S-C₁₀S-C₁ | √ | - | 5.0 | 87.6 | 17.5 | 92.6 |
| C₁-S-C₁₂-S-C₁ | - | 6.0 | 8.0 | 79.6 | 10.0 | 93.6 |
| C₁-S-C₁₂-S-C₁ | √ | - | 5.2 | 84.8 | 16.3 | 90.0 |

| | | | | | | |
|---|---|---|---|---|---|---|
| a) 100 mmol MC, 110 mmol SO₂Cl₂, 4 h, room temperature; | | | | | | |
| b) expressed as the absolute yield of the compounds listed, as determined by quantitative GC; | | | | | | |
| c) sum of MC+OCMC+PCMC; low mass balance indicates either physical loss of material or the production of other materials such as dichloro compounds. | | | | | | |

### Example 2

Table 2 illustrates the effectiveness of various catalysts of general formula (II), with both R³ and R⁴ being n-butyl groups, each R⁵ having a different chain length, in both the absence and presence of AlCl₃. The para:ortho ratios generally increase with the length of the R⁵ group, and the best overall results (including a high yield of desired product) are achieved with C₁₀ to C₁₂.

**Table 2**

| Chlorination of *meta*-cresol in the presence of dithiaalkanes^{a} | | | | | | |
|---|---|---|---|---|---|---|
| catalyst | presence of AlCl₃ | MC mol% | OCMC mol%^{b} | PCMC mol%^{b} | *p*/*o* ratio | *mass* balance % ^{c} |
| C₄-S-C₄-S-C₄ | - | 11.8 | 14.2 | 67.3 | 4.7 | 93.3 |
| C₄-S-C₄-S-C₄ | √ | 9.0 | 8.8 | 64.0 | 7.2 | 81.8 |
| C₄-S-C₅-S-C₄ | - | 6.7 | 10.2 | 83.1 | 8.1 | 100.0 |
| C₄-S-C₅-S-C₄ | √ | 0.6 | 6.5 | 86.8 | 13.4 | 93.9 |
| C₄-S-C₆-S-C₄ | - | 5.4 | 9.6 | 77.6 | 8.1 | 92.6 |
| C₄-S-C₆-S-C₄ | √ | 7.2 | 6.0 | 78.8 | 13.1 | 92.0 |
| C₄-S-C₇-S-C₄ | - | 4.0 | 8.8 | 85.0 | 9.7 | 97.8 |
| C₄-S-C₇-S-C₄ | √ | 10.6 | 3.2 | 72.4 | 22.7 | 86.2 |
| C₄-S-C₈-S-C₄ | - | 9.6 | 6.8 | 76.8 | 11.3 | 93.2 |
| C₄-S-C₈-S-C₄ | √ | 13.2 | 4.8 | 72.2 | 15.1 | 90.2 |
| C₄-S-C₁₀-S-C₄ | - | 5.2 | 7.0 | 84.6 | 12.1 | 96.8 |
| C₄-S-C₁₀-S-C₄ | √ | 1.8 | 4.8 | 92.8 | 19.3 | 99.4 |
| C₄-S-C₁₂-S-C₄ | - | 3.5 | 6.9 | 89.6 | 13.0 | 100.0 |
| C₄-S-C₁₂-S-C₄ | √ | - | 4.4 | 91.8 | 20.9 | 96.2 |

| | | | | | | |
|---|---|---|---|---|---|---|
| a) 100 mmol MC, 110 mmol SO₂Cl₂, 4 h, room temperature; | | | | | | |
| b) expressed as the absolute yield of the compounds listed, as determined by quantitative GC; | | | | | | |
| c) sum of MC+OCMC+PCMC; low mass balance indicates either physical loss of material or the production of other materials such as dichloro compounds. | | | | | | |

### Example 3

Table 3 below illustrates the effectiveness of various catalysts of general formula (II), with R⁵ being a straight chain C₁₂ group, R³ and R⁴ being a series of straight chain alkyl groups each with a different chain length, in both the absence and presence of AlCl₃. The best overall results are obtained using C₄-S-C₁₂-S-C₄ as the catalyst.

**Table 3**

| Chlorination of *meta*-cresol in the presence of dithiaalkanes^{a} | | | | | | |
|---|---|---|---|---|---|---|
| catalyst | presence of AlCl₃ | MC mol% | OCMC mol% ^{b} | PCMC mol%^{b} | *p*/*o* ratio | mass balance %^{c} |
| C₁-S-C₁₂-S-C₁ | - | 6.0 | 8.0 | 79.6 | 10.0 | 93.6 |
| C₁-S-C₁₂-S-C₁ | √ | - | 5.2 | 84.8 | 16.2 | 90.0 |
| C₂-S-C₁₂-S-C₂ | - | 9.0 | 7.2 | 76.0 | 10.5 | 92.2 |
| C₂-S-C₁₂-S-C₂ | √ | 4.0 | 5.8 | 67.6 | 11.5 | 77.4 |
| C₃-S-C₁₂-S-C₃ | - | 9.4 | 6.6 | 84.0 | 12.7 | 100.0 |
| C₃-S-C₁₂-S-C₃ | √ | 5.4 | 4.8 | 82.8 | 17.4 | 93.0 |
| C₄-S-C₁₂-S-C₄ | - | 3.5 | 6.9 | 89.6 | 13.0 | 100.0 |
| C₄-S-C₁₂-S-C₄ | √ | - | 4.4 | 91.8 | 20.7 | 96.2 |
| C₅-S-C₁₂-S-C₅ | - | 6.4 | 7.4 | 79.2 | 10.7 | 93.0 |
| C₅-S-C₁₂-S-C₅ | √ | 0.5 | 5.2 | 85.0 | 16.8 | 90.7 |
| C₆-S-C₁₂-S-C₆ | - | 0.5 | 6.6 | 82.2 | 12.4 | 89.3 |
| C₆-S-C₁₂-S-C₆ | √ | 7.6 | 5.0 | 70.7 | 14.2 | 83.3 |
| C₇-S-C₁₂-S-C₇ | - | 0.6 | 7.2 | 83.2 | 11.6 | 91.0 |
| C₇-S-C₁₂-S-C₇ | √ | 8.8 | 6.0 | 77.4 | 12.9 | 92.2 |

| | | | | | | |
|---|---|---|---|---|---|---|
| a) 100 mmol MC, 110 mmol SO₂Cl₂, 4 h, room temperature; | | | | | | |
| b) expressed as the absolute yield of the compounds listed, as determined by quantitative GC; | | | | | | |
| c) sum of MC+OCMC+PCMC; low mass balance indicates either physical loss of material or the production of other materials such as dichloro compounds. | | | | | | |

### Example 4

This example (see Table 4 below) illustrates the effect of changing the Lewis acid present in the reaction mixture, where the catalyst is C₄-S-C₁₂-S-C₄. As can be seen, the most preferred co-catalyst is AlCl₃, though both FeCl₃ and ZnCl₂ perform well.

**Table 4**

| Lewis acid | OCMC mol% | MC mol% | PCMC mol% | p/o ratio | mass balance % |
|---|---|---|---|---|---|
| AlCl₃ | 4.6 | - | 95.4 | 20.7 | 96.2 |
| FeCl₃ | 5.5 | 11.3 | 83.2 | 14.8 | 94.0 |
| ZnCl₂ | 5.8 | 11.7 | 82.5 | 14.0 | 89.0 |
| 3x ZnCl₂ | 5.6 | 0.7 | 93.7 | 17.0 | 89.2 |
| N B. The mol% values given in Table 4 are expressed as proportional amounts of the product mixture (ie their sum is 100%), rather than (as in the other Tables) as absolute yields (whose sum is that of the % mass balance). | | | | | |

### Example 5

This Example illustrates the use of an exemplary catalyst of general formula II according to the invention, of the schematic formula C₄-S-C₁₂-S-C₄ (where the C₄ groups are n-butyl groups), in the chlorination of a variety of substrates within the scope of application of the invention. The results are given in Table 5 below.

**Table 5**

| Chlorination of various substrates using C₄-S-C₁₂-S-C₄ as a catalyst in the presence of AlCl₃^{a} | | | | | | |
|---|---|---|---|---|---|---|
| substrate | starting mat. mol%^{b} | *ortho*-chloro-comp. mol%^{b} | *para*-chloro-comp. mol%^{b} | *p*/*o* ratio | temp. °C | mass balance %^{c} |
| phenol | 11.4 | 6.6 | 76.4 | 11.5 | 35 | 94.4 |
| anisole | 0.9 | 10.8 | 88.3 | 8.2 | 20 | 100 |
| m-xylenol | 22.2 | 7.2 | 44.4 | 6.1 | 70 | 73.8 |

| | | | | | | |
|---|---|---|---|---|---|---|
| a) 100 mmol substrate, 110 mmol SO₂Cl₂, 2.7 mmol C₄-S-C₁₂-S-C₄, 3.8 mmol AlCl₃, 4 h, | | | | | | |
| b) expressed as the absolute yield of the compounds listed, as determined by quantitative GC; | | | | | | |
| c) sum of compounds listed; low mass balance indicates either physical loss of material or the production of other materials such as dichloro compounds. | | | | | | |

## Claims

1. A process for the chlorination of an aromatic compound of the following formula (A): 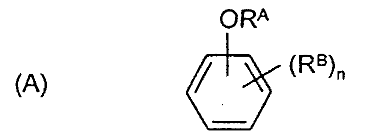 wherein R^{A} is H or C₁₋C₁₂ alkyl, cycloalkyl, aryl, alkaryl, aralkyl or carboxyalkyl, the or each R^{B} independently is selected from H, C₁₋C₄ alkyl, C₁₋C₄ haloalkyl, C₁₋C₄ alkoxy, C₅₋C₁₂ aryl, alkaryl or aralkyl, or halogen, n is an integer which is 0, 1 or 2, and the or each R^{B}, if present, may independently be attached at the ortho or the meta position, the said process comprising reacting the aromatic compound with a chlorinating agent in the presence of a sulphur-containing catalyst, optionally also in the presence of a Lewis acid co-catalyst of the formula MXₘ, where: M is a metal or metalloid; X is an electronegative group; and m is an integer;
**characterised in that** the sulphur-containing catalyst is a compound according to the following formula (II): 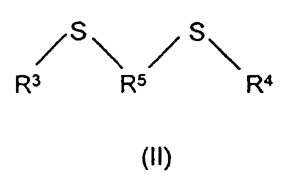 in which:
R³ and R⁴ are each independently selected from the group consisting of: H, optionally substituted straight or branched chain alkyl or alkanediyl having from 1 to 20 carbon atoms, optionally substituted straight or branched chain alkaryl or aralkyl having from 5 to 20 carbon atoms, and optionally substituted aryl having from 5 to 20 carbon atoms; and
R⁵ is an optionally substituted straight or branched chain alkylene, arylene, alkarylene or arylalkylene group having from 4 to 20 carbon atoms;
provided that when R⁵ is a C₄ alkylene group,a Lewis acid co-catalyst of the formula MXₘ (as defined above) is present.

2. A process according to claim 1, wherein, in the formula (A), R^{A} is hydrogen.

3. A process according to claim 1 or claim 2, wherein, in the formula (A), n is 1 and the group R^{B} is in a position meta to the group OR^{A}.

4. A process according to claim 1, wherein the aromatic compound is selected from phenol, anisole, m-cresol and m-xylenol.

5. A process according to claim 4, wherein the aromatic compound is phenol.

6. A process according to any preceding claim, wherein the chlorinating agent is sulphuryl chloride.

7. A process according to any preceding claim, wherein the chlorination reaction is carried out in a homogenous liquid phase, without the presence of added solvent.

8. A process according to any preceding claim, wherein the chlorination reaction is carried out at a temperature of below 35°C.

9. A process according to claim 8, wherein the chlorination reaction is carried out at a temperature of from 15 to 30°C .

10. Use of a sulphur-containing compound of formula (II) as defined in claim 1, wherein R³, R⁴ and R⁵ therein have the same meanings as in claim 1, as a catalyst in the chlorination of an aromatic compound according to formula (A) as defined in claim 1, wherein R^{A}, R^{B} and n therein have the same meanings as in claim 1, by a chlorinating agent.

11. Use according to claim 10, of a compound of formula (II) as defined in claim 1, wherein R⁵ is an unbranched, unsubstituted alkanediyl group containing from 4 to 12 carbon atoms.

12. Use according to claim 10, of a compound of formula (II) as defined in claim 1, wherein the groups R³ and R⁴ have the same carbon atom framework and both groups are unbranched and unsubstituted and independently contain from 1 to 7 carbon atoms.

13. Use according to claim 10, of a compound of formula (II) as defined in claim 1, wherein the groups R³ and R⁴ have different carbon atom frameworks, R³ being selected from the group consisting of: H, optionally substituted straight or branched chain alkyl or alkanediyl having from 1 to 20 carbon atoms and optionally substituted aryl having from 5 to 20 carbon atoms, and R⁴ is different from R³ and is selected from the group consisting of:
optionally substituted straight or branched chain alkyl or alkanediyl having from 1 to 20 carbon atoms and
optionally substituted straight or branched chain alkaryl or aralkyl having from 5 to 20 carbon atoms.

14. A dithiaalkane compound which is:
2,10-dithiaundecane
2,15-dithiahexadecane
5,13-dithiaheptadecane
5,14-dithiaoctadecane
5,18-dithiadocosane
6,13-dithiaoctadecane
8,15-dithiadocosane
3,16-dithiaoctadecane
4,17-dithiaeicosane
6,19-dithiatetracosane
7,20-dithiahexacosane
8,21-dithiaoctacosane
2,7-dithiaundecane
2,2-dimethyl-3,8-dithiadodecane
1-phenyl-2,7-dithiaundecane
1-phenyl-1,6-dithiadecane
1-phenyl-8-methyl-1,6-dithianonane
1,7-diphenyl-1,6-dithiaheptane
5,12-dithianonadecane
1-phenyl-2,9-dithiatridecane

## Patentansprüche

1. Verfahren zur Chlorierung einer aromatischen Verbindung der folgenden Formel (A): 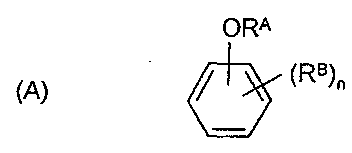 worin R^{A} H oder C₁-C₁₂-Alkyl, Cycloalkyl, Aryl, Alkaryl, Aralkyl oder Carboxyalkyl ist, das oder jedes R⁸ jeweils unabhängig von den anderen aus H, C₁-C₄-Alkyl, C₁-C₄-Haloalkyl, C₁-C₄-Alkoxy, C₅-C₁₂-Aryl, -Alkaryl oder -Aralkyl oder Halogen ausgewählt ist, n eine ganze Zahl ist, die 0, 1 oder 2 ist, und das oder jedes R^{B}, falls vorhanden, jeweils unabhängig von den anderen an die ortho- oder die metha-Position gebunden sein kann, wobei das Verfahren das Umsetzen der aromatischen Verbindung mit einem Chlorierungsmittel in Gegenwart eines schwefelhältigen Katalysators umfasst, gegebenenfalls auch in Gegenwart eines Lewissäure-Cokatalysators der Formel MXₘ, worin: M ein Metall oder Metalloid ist; X eine elektronegative Gruppe ist; und m eine ganze Zahl ist;
**dadurch gekennzeichnet, dass** der schwefelhältige Katalysator eine Verbindung der folgenden Formel (II): 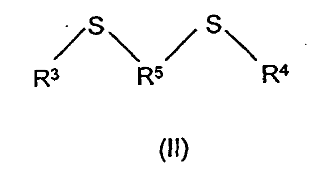 ist, worin:
R³ und R⁴ jeweils unabhängig voneinander aus der Gruppe ausgewählt sind, die besteht aus: H, gegebenenfalls substituiertem, unverzweigtem oder verzweigtem Alkyl oder Alkandiyl mit 1 bis 20 Kohlenstoffatomen, gegebenenfalls substituiertem, unverzweigtem oder verzweigtem Alkaryl oder Aralkyl mit 5 bis 20 Kohlenstoffatomen, und gegebenenfalls substituiertem Aryl mit 5 bis 20 Kohlenstoffatomen; und
R⁵ eine gegebenenfalls substituierte unverzweigte oder verzweigte Alkylen-, Arylen-, Alkarylen- oder Arylalkylengruppe mit 4 bis 20 Kohlenstoffatomen ist;
mit der Maßgabe, dass, wenn R⁵ eine C₄-Alkylengruppe ist, ein Lewissäure-Cokatalysator der Formel MXₘ (wie oben definiert) vorhanden ist.

2. Verfahren nach Anspruch 1, worin in Formel (A) R^{A} Wasserstoff ist.

3. Verfahren nach Anspruch 1 oder 2, worin in Formel (A) n = 1 ist und sich die Gruppe R^{B} in meta-Position zur Gruppe OR^{A} befindet.

4. Verfahren nach Anspruch 1, worin die aromatische Verbindung aus Phenol, Anisol, m-Kresol und m-Xylenol ausgewählt ist.

5. Verfahren nach Anspruch 4, worin die aromatische Verbindung Phenol ist.

6. Verfahren nach einem der vorangegangenen Ansprüche, worin das Chlorierungsmittel Sulfurylchlorid ist.

7. Verfahren nach einem der vorangegangenen Ansprüche, worin die Chlorierungsreaktion in einer homogenen flüssigen Phase ohne Zusatz von Lösungsmittel durchgeführt wird.

8. Verfahren nach einem der vorangegangenen Ansprüche, worin die Chlorierungsreaktion bei einer Temperatur unterhalb von 35 °C durchgeführt wird.

9. Verfahren nach Anspruch 8, worin die Chlorierungsreaktion bei einer Temperatur von 15 bis 30 °C durchgeführt wird.

10. Verwendung einer schwefelhältigen Verbindung der Formel (II), wie in Anspruch 1 definiert, worin R³, R⁴ und R⁵ darin die gleiche Bedeutung wie in Anspruch 1 haben, als Katalysator bei der Chlorierung einer aromatischen Verbindung der Formel (A), wie in Anspruch 1 definiert, worin R^{A}, R^{B} und n darin die gleiche Bedeutung wie in Anspruch 1 haben, mit einem Chlorierungsmittel.

11. Verwendung nach Anspruch 10 einer Verbindung der Formel (II), wie in Anspruch 1 definiert, worin R⁵ eine unverzweigte unsubstituierte Alkandiylgruppe ist, die 4 bis 12 Kohlenstoffatome enthält.

12. Verwendung nach Anspruch 10 einer Verbindung der Formel (II), wie in Anspruch 1 definiert, worin die Gruppen R³ und R⁴ das gleiche Kohlenstoffgerüst aufweisen und beide Gruppen unverzweigt und unsubstituiert sind und unabhängig voneinander 1 bis 7 Kohlenstoffatome enthalten.

13. Verwendung nach Anspruch 10 einer Verbindung der Formel (II), wie in Anspruch 1 definiert, worin die Gruppen R³ und R⁴ unterschiedliche Kohlenstoffgerüste aufweisen, wobei R³ aus der Gruppe ausgewählt ist, die aus Folgenden besteht: H, gegebenenfalls substituiertem, unverzweigtem oder verzweigtem Alkyl oder Alkandiyl mit 1 bis 20 Kohlenstoffatomen und gegebenenfalls substituiertem Aryl mit 5 bis 20 Kohlenstoffatomen, und R⁴ von R³ verschieden ist und aus der aus Folgenden bestehenden Gruppe ausgewählt ist: gegebenenfalls substituiertem, unverzweigtem oder verzweigtem Alkyl oder Alkandiyl mit 1 bis 20 Kohlenstoffatomen und gegebenenfalls substituiertem, unverzweigtem oder verzweigtem Alkaryl oder Aralkyl mit 5 bis 20 Kohlenstoffatomen.

14. Dithialkanverbindung, die Folgendes ist:
2,10-Dithiaundecan
2,15-Dithiahexadecan
5,13-Dithiaheptadecan
5,14-Dithiaoctadecan
5,18-Dithiadocosan
6,13-Dithiaoctadecan
8,15-Dithiadocosan
3,16-Dithiaoctadecan
4,17-Dithiaeicosan
6,19-Dithiatetracosan
7,20-Dithiahexacosan
8,21-Dithiaoctacosan
2, 7-Dithiaundecan
2,2-Dimethyl-3,8-dithiadodecan
1-Phenyl-2,7-dithiaundecan
1-Phenyl-1,6-dithiadecan
1-Phenyl-8-methyl-1,6-dithianonan
1,7-Diphenyl-1,6-dithiaheptan
5,12-Dithianonadecan
1-Phenyl-2,9-dithiatridecan.

## Revendications

1. Procédé pour la chloruration d'un composé aromatique de la formule (A) qui suit: 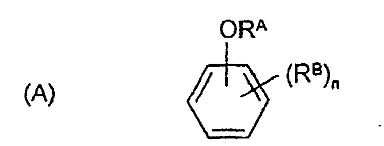 où R^{A} est H ou alkyle C₁-C₁₂, cycloalkyle, aryle, alkaryle, aralkyle ou carboxyalkyle, le ou chaque R^{B} est indépendamment sélectionné parmi H, alkyle C₁-C₄, haloalkyle C₁-C₄, alcoxy C₁-C₄, aryle C₅-C₁₂, alkaryle ou aralkyle ou halogène, n est un entier qui est 0, 1 ou 2, et le ou chaque R^{B}, s'il est présent, peut indépendamment être attaché à la position ortho ou méta, ledit procédé comprenant la réaction du composé aromatique avec un agent de chloruration en présence d'un catalyseur contenant du soufre, facultativement également en présence d'un co-catalyseur d'acide de Lewis de la formule MXₘ, où: M est un métal ou métalloïde; X est un groupe électronégatif; et m est un entier;
**caractérisé en ce que** le catalyseur contenant du soufre est un composé selon la formule (II) qui suit: 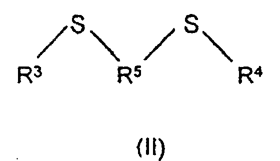 dans laquelle:
chacun de R³ et R⁴ est indépendamment sélectionné dans le groupe consistant en: H, alkyle ou alkanediyle facultativement substitué à chaîne droite ou ramifiée ayant de 1 à 20 atomes de carbone, alkaryle ou aralkyle facultativement substitué à chaîne droite ou ramifiée ayant de 5 à 20 atomes de carbone et aryle facultativement substitué ayant de 5 à 20 atomes de carbone; et
R⁵ est un groupe alkylène, arylène, alkarylène ou arylalkylène facultativement substitué à chaîne droite ou ramifiée ayant de 4 à 20 atomes de carbone;
à condition que quand R⁵ est un groupe alkylène C₄, un co-catalyseur d'acide de Lewis de la formule MXₘ (telle que définie ci-dessus) soit présent.

2. Procédé selon la revendication 1, où, dans la formule (A), R^{A} est hydrogène.

3. Procédé selon la revendication 1 ou la revendication 2, où, dans la formule (A), n est 1 et le groupe R^{B} est en une position méta au groupe OR^{A}.

4. Procédé selon la revendication 1, où le composé aromatique est sélectionné parmi phénol, anisole, m-crésol et m-xylénol.

5. Procédé selon la revendication 4, où le composé aromatique est phénol.

6. Procédé selon toute revendication précédente, où l'agent de chloruration est le chlorure de sulfuryle.

7. Procédé selon toute revendication précédente, où la réaction de chloruration est effectuée dans une phase liquide homogène, sans la présence d'un solvant ajouté.

8. Procédé selon toute revendication précédente, où la réaction de chloruration est effectuée à une température de moins de 35°C.

9. Procédé selon la revendication 8, où la réaction de chloruration est effectuée à une température de 15 à 30°C.

10. Utilisation d'un composé contenant du soufre de la formule (II) telle que définie à la revendication 1, où R³, R⁴ et R⁵ ont les mêmes significations qu'à la revendication 1, comme catalyseur dans la chloruration d'un composé aromatique selon la formule (A) telle que définie à la revendication 1, où R^{A}, R^{B} et n ont les mêmes significations qu'à la revendications 1, par un agent de chloruration.

11. Utilisation selon la revendication 10 d'un composé de la formule (II) telle que définie à la revendication 1, où R⁵ est un groupe alcanediiyle non ramifié non substitué contenant de 4 à 12 atomes de carbone.

12. Utilisation selon la revendication 10 d'un composé de la formule (II) telle que définie à la revendication 1, où les groupes R³ et R⁴ ont la même charpente d'atomes de carbone et les deux groupes sont non ramifiés et non substitués et contiennent indépendamment de 1 à 7 atomes de carbone.

13. Utilisation selon la revendication 10 d'un composé de la formule (II) telle que définie à la revendication 1, où les groupes R³ et R⁴ ont différentes charpentes d'atomes de carbone, R³ étant sélectionné dans le groupe consistant en: H, alkyle ou alcanediiyle facultativement substitué à chaîne droite ou ramifiée ayant de 1 à 20 atomes de carbone et aryle facultativement substitué ayant de 5 à 20 atomes de carbone, et R⁴ est différent de R³ et est sélectionné dans le groupe consistant en: alkyle ou alcanediiyle facultativement substitué à chaîne droite ou ramifiée ayant de 1 à 20 atomes de carbone et alkaryle ou aralkyle facultativement substitué à chaîne droite ou ramifiée ayant de 5 à 20 atomes de carbone.

14. Composé de dithiaalcane qui est:
2,10-dithiaundécane
2,15-dithiahexadécane
5,13-dithiaheptadécane
5,14-dithiaoctadécane
5,18-dithiadocosane
6,13-dithiaoctadécane
8,15-dithiadocosane
3,16-dithiaoctadecane
4,17-dithiaéicosane
6,19-dithiatétracosane
7,20-dithiahexacosane
8,21-dithiaoctacosane
2,7-dithiaundécane
2,2-diméthyl-3,8-dithiadodécane
1-phényl-2,7-dithiaundécane
1-phényl-1,6-dithiadécane
1-phényl-8-méthyl-1,6-dithianonane
1,7-diphényl-1,6-dithiaheptane
5,12-dithianonadécane
1-phényl-2,9-dithiatridécane
